# DEMANDE DE BREVET EUROPEEN

(11) **EP 2 359 838 A1**
(43) Date de publication de la demande: **24.08.2011**
(21) Numéro de dépôt: 11305102.3
(22) Date de dépôt: 01.02.2011
(51) Int. Cl.: A61K 35/74, A23L 1/30, A61P 3/04

(54) **Préparation destinée à traiter l`excès pondéral et les désordres associés et applications de ladite préparation**

(30) Priorité: 02.02.2010 FR 1050734
(71) Demandeur: Aragan, 75008 Paris (FR)
(72) Inventeur: Arampatis, Charalampos, 06000, NICE (FR)
(74) Mandataire: Demoly, Pascale

(57) **Abrégé**

Préparation destinée à corriger une dysbiose intestinale au niveau du côlon, comportant au moins comme premier probiotique des bifidobactéries sous forme vivante, et éventuellement un autre probiotique. Dans le but d'assurer un taux de survie et d'adhérence spécifique des bifidobactéries au niveau du côlon, la concentration de la préparation en bifidobactéries est supérieure à 3 milliards de bifidobactéries par gramme de préparation, et les bifidobactéries sont doublement encapsulées, de manière à conserver leur intégrité au cours du transfert dans le tractus gastro-intestinal.

La préparation peut être un médicament, un complément alimentaire, un aliment, un additif alimentaire, ou un aliment destiné à des fins médicales spéciales

Application notamment au traitement de l'excès pondéral et de ses désordres associés, chez l'adolescent et l'adulte.

## Description

La présente invention se situe dans le domaine du traitement, par une modulation du microbiote intestinal, de l'obésité, de l'excès pondéral, du diabète de type II, de l'hypercholestérolémie, et des désordres métaboliques associés.

Dans le domaine général de la composition du microbiote intestinal, déterminée sur la base du séquençage de l'ARN 16S ribosomal, on sait qu'elle est représentée à plus de 90% par deux grands phyla, le phylum des firmicutes et celui des bacteroïdètes (Steven R. Gill., et al, Science 2006 June 2 ;312 : 1355-1359). Les firmicutes contiennent entre autres les espèces de lactobacilles, alors que les bactéroïdètes sont représentées par les espèces de bactéroïdes. Parmi les autres phyla composant le microbiote intestinal, celui des actinobactéries est qualitativement important, en particulier par la représentation en bifidobactéries (Comparative Analysis of Human Gut Microbiota by Barcoded Pyrosequencing, Anders F. Andersson, Mathilda Lindberg, Hedvig Jakobsson, Fredrik Bäckhed, Pål Nyrén, and Lars Engstrand, PLoS ONE, v.3(7); 2008). Ce microbiote, considéré aujourd'hui comme un organe à part entière, est impliqué dans des fonctions bien connues : par exemple, digestion de résidus pour produire des métabolites assimilables, exclusion de l'installation de bactéries pathogènes, stimulation de l'immunité, synthèse des vitamines B12 et K. Pour Delzenne et Cani (Medecine/Sciences, 2008 ;24 :505-10), les 10¹⁴ bactéries qui la composent constituent un biotope spécifique à chaque individu, lequel peut alors être considéré comme un métagénome réunissant son génome humain et la somme des génomes bactériens présents dans son tractus gastro-intestinal.

Dans le domaine général du traitement de l'obésité, de l'excès pondéral et des désordres associés, on connaît déjà des preuves de l'implication de la flore intestinale sur l'obésité. En particulier, Turnbaugh et al. (An obesity-associated gut microbiome with increased capacity for energy harvest. Nature 2006 ;444 :1027-31) ont transféré le microbiote de souris obèses sur des souris minces axéniques, dont le poids s'est alors mis à augmenter en deux semaines.

Le document WO-200924429 cible des protéobactéries intestinales, et plus particulièrement des enterobactéries Gram-négatives et des deferribactères, pour obtenir une diminution pondérale. Or, Cani et al. (Changes in gut microbiota control metabolic endotoxemia-induced inflammation in high-fat diet-induced obesity and diabetes in mice. Diabetes 2008; 57: 1470-81) ont montré qu'une augmentation de la population de bactéries Gram-négative portant un lipopolysaccharide (LPS) peut induire un état inflammatoire. Ce LPS est produit par la lyse des bactéries, et stimule après absorption la sécrétion de cytokines pro-inflammatoires qui participent à l'installation du diabète de type II en stimulant l'insulino-résistance (Vreugdenhil AC, Rousseau CH, Hartung T et al. Lipopolysaccharide (LPS)-binding protein mediates LPS detoxification by chylomicrons. J Immunol 2003 ; 170 : 1399-405). Un traitement par antibiotique permet alors de limiter le taux de LPS et l'inflammation associée (Cani et al., supra). Ces résultats sont corrélés avec ceux de Creely et al (Creely SJ, McTernan PG, Kusminski CM, et al. Lipopolysaccharide activates an innate immune system response in human adipose tissue in obesity and type 2 diabetes. Am J Physiol Endocrinol Metab 2007; 292 : E740-7) qui montrent qu'une alimentation riche en lipides modifie la composition de la flore intestinale en favorisant les populations bactériennes à LPS et donc les risques d'inflammation et de diabète de type II. Le traitement décrit dans WO-200924429 permet donc de diminuer l'inflammation liée à l'obésité. Ce document propose différents effecteurs pour diminuer les populations bactériennes visées : utilisation de bactériophages T4 ou T7, utilisation de levures saccharomyces, traitements par antibiotiques, ou par composés phytochimiques. Mais l'inconvénient est qu'il permet surtout de diminuer spécifiquement la présence de bactéries impliquées dans les phénomènes inflammatoires, mais pas de rétablir l'équilibre global de la flore intestinale. Par ailleurs, la présence dans un produit alimentaire des effecteurs utilisés n'est pas souhaitable.

Pour résoudre ces inconvénients, il a déjà été proposé de recourir aux probiotiques ou aux prébiotiques.

Ainsi, les probiotiques sont des bactéries vivantes, administrées par voie orale à l'homme ou à l'animal, qui présentent un effet bénéfique pour la santé ou le bien être du sujet en améliorant le microbiote intestinal (Fuller, R., probiotics in man and animals, 1989, J. Appl. Microbiol., 66 : 365-378). Les prébiotiques sont des composés alimentaires non digestibles qui sont dégradés par la flore intestinale. Ils ont des effets bénéfiques sur l'hôte, d'abord du fait de la stimulation de la croissance des bactéries intestinales favorables qu'ils induisent, ensuite parce qu'ils peuvent stimuler spécifiquement la synthèse par ces bactéries de nutriments utiles pour l'organisme (Gibson GR, Roberfroid MB. Dietary modulation of the human colonic microbiota: introducing the concept of prebiotics. J Nutr 1995; 125 : 1401-12).

L'ingestion de probiotiques par l'organisme ne supprime pas le besoin de nutriments directement absorbables dans l'alimentation. Pour résoudre ce problème, le brevet US-2008286252 prévoit que la digestion des prébiotiques par les probiotiques est réalisée en fermenteur, et ce sont les produits issus de cette digestion qui sont fournis comme préparation à visée alimentaire.

L'inconvénient de cette solution est qu'elle ne vise pas à moduler le microbiote intestinal. Pour pallier cet inconvénient, on doit faire ingérer par le sujet des probiotiques ou des prébiotiques.

On apprend par Ito et al. (Effect of administration of galactooligosaccharides on the human faecal microflora, stool weight and abdominal sensation. Microb. Ecol. Health Dis. 1990. 3 :285-292) que les galactooligosaccharides (GOS) sont des prébiotiques bifidogènes : ils vont stimuler la croissance des bifidobactéries dans l'intestin. On connaît une autre application du GOS par le brevet FR-2844453 qui montre qu'un traitement par ce prébiotique bifidogène, de sujets ayant une tendance à l'obésité, aide à prévenir le diabète de type II. La capacité bifidogénique d'autres prébiotiques comme les oligofructoses, les fructo-oligosachharides (FOS) et les trans-galacto-oligosaccharides (TOS), a été comparée par Bouhnik et al. (The capacity of nondigestible carbohydrates to stimulate fecal bifidobacteria in healthy humans : a double-blind, randomized, placebo-controlled, parallel-group, dose-response relation study. Ann. J. Clin. Nutr. 2004 ;80 :1658-64). Par exemple, une alimentation riche en fibres fermentescibles non digestibles, et en particulier riche en fructanes, contribue à diminuer le poids corporel tout en augmentant la population de bifidobactéries qui utilisent préférentiellement ces composés (Cani PD, Joly E, Horsmans Y, et al. Oligofructose promotes satiety in healthy human: a pilot study. Eur J Clin Nutr 2006; 60 : 567-72).

Ces observations sont confirmées par des études cliniques menées sur des adolescents par Abrams et al. (Effect of bifidogene prebiotic supplementation intake on body mass index. J. Pediatr. 2007 Sept. 151 (3) :293-8). Cette étude a montré que les adolescents traités par prébiotique présentaient un poids en moyenne 30% inférieur, une masse graisseuse inférieure de 68% et un IMC (Index de Masse Corporelle) inférieur de 45%. Des résultats menant à des conclusions similaires ont été obtenus en administrant un probiotique comportant des lactobacilles et des bifidobactéries à des femmes enceintes en Finlande (ECO 2009, 17th European Congress on Obesity, Amsterdam 6-9 may 2009).

On sait aussi qu'une augmentation du contenu microbiotique en bifidobactéries s'accompagne d'une diminution des phénomènes inflammatoires liés à l'obésité et au diabète (Cani PD, Neyrinck AM, Fava F, et al. Selective increases of bifidobacteria in gut microflora improve high-fat-diet-induced diabetes in mice through a mechanism associated with endotoxaemia. Diabetologia 2007; 50 : 2374-83). Cet effet est corrélé à un contrôle de l'augmentation du taux de lipopolysaccharides (LPS), dont nous avons vu l'action sur le processus inflammatoire et le déclenchement du diabète. A l'opposé, un régime riche en graisses provoque une diminution de la présence des bifidobactéries, corrélée à une augmentation de l'inflammation et à une perméabilité plus importante de la muqueuse intestinale. Ces désordres sont désignés sous le terme d'endotoxémie.

On apprend également du document WO-2009071086 que l'ingestion de graisses par la paroi intestinale est régulée sous l'effet de facteurs fournis par des lactobacilles ou des bifidobactéries. En effet, le rapport entre flore intestinale et métabolisme des graisses a été démontré par Backhed et al (Backhed F, Ding H, Wang T, et al. The gut microbiota as an environmental factor that regulates fat storage. Proc Natl Acad Sci USA 2004; 101 : 15718-23) via une inactivation de l'expression d'un facteur intestinal, le FIAF (Fasting-induced adipose factor). Le FIAF génère une augmentation de l'activité de l'enzyme lipoprotein lipase (LPL), et in fine la plus grande captation des acides gras par les muscles et le tissu adipeux. Le brevet WO-2009071086 repose donc sur une substance médiatrice de l'expression du FIAF, produite par les probiotiques.

Non seulement la présence accrue de bifidobactéries permet, par phénomène de compétition, de diminuer l'activité des bactéries gram-négatives responsables de la production de LPS, mais aussi, cette présence permet de rétablir l'intégrité de la paroi intestinale. Ce rétablissement s'effectue par une stimulation de la production d'une hormone impliquée dans la croissance et la prolifération de l'épithélium intestinal, le glucagon-like peptide-2 (GLP-2), qui protège ainsi des micro-perforations de la paroi intestinale (Changes in gut microbiota control inflammation in obese mice through a mechanism involving GLP-2-driven improvement of gut permeability. Cani et al., Gut, 23 fev 2009). Le rétablissement de la paroi intestinale permet de prévenir l'absorption des facteurs déclenchant l'inflammation, mais elle réduit aussi de manière globale l'absorption excessive de nutriments. Une action réparatrice de la paroi intestinale peut également être obtenue avec certains nutriments. On apprend en effet de Amasheh et al. (Barrier effects of nutritional factors. Ann. NY Acad. Sci. 2009 May ;1165 :267-73) que des facteurs nutritionnels peuvent agir sur l'état de la barrière intestinale, et en particulier sur l'inflammation intestinale. On sait par ailleurs par Wang et al. (Effect of glutamine on intestinal mucosal barrier function in rats with acute hepatic injury, Zhonghua Shi Yan He Lin Chuang Bing Du Xue Za Zhi. 2009 Feb;23(1):53-5) que la L-Glutamine aide à réparer la muqueuse intestinale. On sait enfin par Yousheng Li et al. (Oral glutamine ameliorates chemotherapy-induced changes of intestinal permeability and does not interfere with the antitumor effect of chemotherapy in patients with breast cancer: A prospective randomized trial. Tumori. 2006, vol. 92, no5, pp. 396-401), que la L-Glutamine agit en contribuant à diminuer la perméabilité de la barrière intestinale dans des situations pathologiques.

Par ailleurs, la synthèse d'autres peptides endogènes est stimulée par les produits de la dégradation des fructanes par les bactéries lactiques : il s'agit de peptides intestinaux impliqués dans la gestion de l'appétit, de la sécrétion et de la sensibilité à l'insuline, de la glycémie et du poids corporel, comme le glucagon-like peptide 1 (GLP-1) ou le glucose-dependent insulinotropic peptide (GIP) (Cani et al., supra).

Pour agir en même temps sur la prise de poids, les phénomènes inflammatoires et le déclenchement du diabète de type II, un traitement doit donc avoir un effet réparateur de la paroi intestinale, qui permet une diminution des phénomènes d'inflammation liés à l'obésité, et favorise la régulation du métabolisme lipidique et celle de l'ingestion des aliments.

Il a déjà été proposé à cette fin d'utiliser des préparations à base de Lactobacilles, combinés optionnellement à des bifidobactéries ou à d'autres bactéries.

Ainsi, le document WO-200743933 nous apprend qu'un probiotique associant des Lactobacillus casei, Lactobacillus acidophilus ou des Bifidobacterium lactis permet de prévenir l'augmentation de poids et l'obésité, d'augmenter la sensation de satiété, de réduire l'ingestion d'aliments, et de réguler les métabolismes glucidique et lipidique.

On connaît aussi, par les documents US-2005186189 et US-7001756 que des souches de Lactobacillus Rhamnosus permettent de réguler le métabolisme du cholestérol et des lipides, et de prévenir les complications associées à l'obésité. Il en est de même dans le brevet WO-2009098355, qui utilise Lactobacillus Rhamnosus pour réguler un profil lipidique qui est associé avec l'inflammation de la muqueuse intestinale. Les brevets EP-1177794 et US-6696057 proposent un traitement de l'obésité et des désordres digestifs par une combinaison de probiotiques, Lactobacillus Bulgaricus et Streptococcus thermophilus.

On sait encore, par le document WO-2007085970, que des souches de Lactobacillus, en présence éventuelle de souches de Bifidobacterium, et en association avec des prébiotiques FOS, GOS et TOS, permet d'agir sur des marqueurs de la satiété, en particuliers des hormones et des neuro-peptides. De même, dans le document WO-2009138448, l'association de Lactobacillus paracasei et de prébiotiques similaires agit via une activation du système nerveux sympathique et de la lipolyse, et une inhibition du système nerveux parasympathique.

On sait encore par le document US-2003166257, que le Lactobacillus salivarius est utilisé comme probiotique en association avec un prébiotique, pour diminuer les phénomènes d'inflammation via son interaction avec une cytokine pro-inflammatoire. On apprend aussi du brevet WO-2009048934 que des souches de lactobacilles et de bifidobactéries ont été associées pour traiter l'état inflammatoire du tractus gastro-intestinal. De manière plus large, le brevet WO-02060276 décrit une association de lactobacilles, propionibactéries, bifidobactéries et prébiotiques bifidogènes dans un objectif de stimulation de l'immunité générale.

De ce qui précède, on peut constater que les lactobacilles sont plus souvent utilisés que les bifidobactéries. WO-2004089115 indique que, pour traiter l'inflammation et d'autres désordres intestinaux, les souches de lactobacilles ont été préférées aux souches de bifidobactéries, car ces dernières sont moins résistantes aux traitements physico-chimiques utilisés dans l'industrie alimentaire. On apprend par ailleurs que certaines souches de Lactobacillus peuvent présenter d'autres propriétés intéressantes.

Ainsi, on sait du document WO-2004144403 qu'une souche de lactobacille synthétise des polysaccharides extra-cellulaires à partir des mono et disaccharides présents dans l'intestin, ce qui réduit donc l'absorption intestinale des glucides. Ainsi, elle joue le rôle de probiotique contre l'obésité et le diabète. De même, dans le brevet US-2008057044, les lactobacilles sélectionnés sont capables de convertir l'acide linoléique et de réduire ainsi la masse graisseuse. On apprend enfin du document WO-2009138092 qu'une stimulation de l'immunité associée à un renforcement de la barrière intestinale est obtenue par l'apport spécifique de probiotiques à base de lactobacilles. Le résultat est obtenu par la production de métabolites spécifiques restaurant la barrière intestinale (Roy CC et al., Short chain fatty acids : ready for prime time ? Nutr. Clin. Pract., 2006; 21 :351-366), et plus particulièrement par la production de polyamines (Lonvaud-Funel A., Biogenic amines in wine : role of acid lactic bacteria. FEMS Microbiol. Letters, 2001, 199 :9-13).

Mais on connaît aussi des inconvénients liés à l'utilisation des lactobacilles, qui font partie du phylum des firmicutes.

On apprend en effet des travaux de Turnbaugh et al (Turnbaugh PJ, Ley RE, Mahowald MA, Magrini V, Mardis ER, Gordon JI. An obesity-associated gut microbiome with increased capacity for energy harvest. Nature 2006; 444 : 1027-31) que le microbiote d'individus obèses récupère plus d'énergie à partir d'une même ration alimentaire que pour des individus sains, conférant ainsi à cette ration alimentaire une plus grande valeur calorique. L'intestin de sujets obèses compte une proportion moindre de bacteroïdètes, et ce sont les firmicutes qui deviennent plus prédominantes. Les firmicutes permettent une meilleure extraction de l'énergie de la ration alimentaire au profit de l'hôte qui les héberge, et participent ainsi à l'excès pondéral du sujet. Un excès énergétique de 1% par rapport aux besoins suffit à générer une augmentation de 20kg du poids corporel sur 20 ans (Hill JO. Understanding and addressing the epidemic of obesity: an energy balance perspective. Endocr Rev 2006 ; 27 : 750-61).

On sait de DRASAR B.S. et BARROW P.A. (Intestinal Microbiology - Aspects of Microbiology 10 - Van Nostrand Reinhold Co. Ltd, U.K. 1985.) que la flore intestinale est répartie topographiquement dans le tractus gastro-intestinal. Pour Rambaud et al. (Flore microbienne intestinale : physiologie et pathologie digestives, Ed. John Libbey, Eurotext), la flore de l'intestin grêle est surtout composée de streptocoques, lactobacilles et entérobactéries. Selon Pierre Bourlioux (Composition et rôles de la flore intestinale, Objectif Nutrition, Institut Danone, n° 41, septembre 1998), la flore dominante du côlon comporte entre autres des bactéroïdètes et des bifidobactéries. Le côlon présente également une flore sous-dominante où apparaissent entre autres des streptocoques, lactobacilles et entérobactéries. On voit donc que pour agir spécifiquement au niveau du côlon, l'utilisation des bifidobactéries est plus indiquée que celle des lactobacilles.

On sait par ailleurs du document WO-200876696, que la proportion entre firmicutes et bacteroïdètes peut constituer un marqueur du risque d'obésité. Il a en effet été montré que le rapport firmicutes/bacteroïdètes est de 10 contre 1 chez un adulte sain, et monte à 100 pour 1 chez un adulte obèse (Ley RE, Turnbaudh PJ, Klein S, Gordon JI. 2006. Microbial ecology : human gutmicrobes associated with obesity. Nature. 444 : 1022-3). Tout en gardant une spécificité propre à chaque individu, le microbiote subit une évolution corrélée au poids de l'individu ou au suivi de régimes amincissants.

Un profil microbiotique de l'obésité ou du risque d'obésité est ainsi défini. Dans ce contexte, même si les effets favorables immédiats de l'utilisation de lactobacilles comme probiotiques sont connus, comme rendu compte précédemment, il peut paraître plus prudent pour le moyen terme de ne pas modifier le rapport firmicutes/bactéroïdètes en faveur des firmicutes. D'ailleurs, cette prudence est renforcée par les observations de Didier Raoult (Probiotics and obesity : a link ?, Nature Reviews Microbiology 7, septembre 2009 : 616) concernant la prise de poids de poulets ayant reçu des probiotiques à base de lactobacilles.

On sait également que l'espèce de Bacteroides thetaiotaomicron, qui représente environ 6% du microbiote humain (Eckburg, P. B., E. M. Bik, C. N. Bernstein, E. Purdom, L. Dethlefsen, M. Sargent, S. R. Gill, K. E. Nelson, and D. A. Relman. 2005. Diversity of the human intestinal microbial flora. Science 308:1635-1638.), fait partie de la famille de Bacteroides et elle est considérée comme une bactérie qui vit en symbiose dans le colon humain et qui contribue à la stabilité et l'équilibre du microbiote humain. Elle est une bactérie commensale de l'Homme, non pathogène, sous condition qu'elle ne sorte pas de sa niche écologique habituelle pour se rendre dans des tissus normalement stériles.

Les espèces de Bacteroides incluent également des bactéries potentiellement pathogènes qui sont responsables des infections intestinales, et identifiées comme les bactéries responsables de troubles intestinaux comme la diarrhée aigue (Nguyen, T. V., P. L. Van, C. L. Huy, K. N. Gia, and A. Weintraub. 2006. Etiology and epidemiology of diarrhea in children in Hanoi, Vietnam. Int. J. Infect. Dis. 10:298-308.)

La majorité des études in vivo ont démontré que l'addition dans l'alimentation humaine de prébiotiques de type oligofructose et inuline n'influence pas la population des espèces des Bacteroides spp. On apprend que les populations de ces dernières ne sont ni stimulées, ni diminuées par l'administration de prébiotiques type oligofructose. (Roberfroid, M. B. 2005. Inulin-type fructans and the modulation of the intestinal microflora, p. 151-181. In M. B. Roberfroid and I. Wolinsky (ed.), Inulin-type fructans: functional food ingredients. CRC Press LCC, Boca Raton, FL.).

Néanmoins, certaines espèces de Bacteroides spp comme par exemple l'espèce de Bacteroides thetaiotaomicron est capable de dégrader des prébiotiques de type oligofructose. (Van der Meulen, R., L. Makras, K. Verbrugghe, T. Adriany, and L. De Vuyst. 2006. In vitro kinetic analysis of oligofructose consumption by Bacteroides and Bifidobacterium spp. indicates different degradation mechanisms. Appl. Environ. Microbiol. 72:1006-1012).

L'invention a donc pour objectif d'améliorer le traitement de l'excès pondéral et des désordres physiologiques associés par une nouvelle préparation qui ne comporte pas de bactéries du phylum des firmicutes et en particulier pas de lactobacilles. Une telle préparation peut être notamment un médicament, un complément alimentaire, un aliment, un additif alimentaire, un aliment destiné à des fins médicales spéciales.

Elle vise en particulier à proposer une nouvelle préparation permettant d'obtenir au moins un effet réparateur de la paroi intestinale.

Elle vise aussi à proposer une préparation particulièrement adaptée au traitement de l'excès pondéral, et/ou de l'obésité, et/ou du diabète de type II, et/ou de l'hypercholestérolémie, notamment chez l'adolescent et l'adulte.

Elle vise également à proposer une préparation qui comporte certaines espèces non pathogènes de la famille de Bacteroides, et plus particulièrement la population de Bacteroides thetaiotaomicron, ou qui augmente sélectivement leur présence.

Elle vise encore à proposer une préparation particulièrement adaptée pour stabiliser le poids chez des sujets qui étaient en surpoids ou obèses, ayant perdu du poids volontairement à la suite d'un régime ou d'une intervention chirurgicale.

Avec ces objectifs en vue, l'invention a pour objet une préparation destinée à corriger une dysbiose intestinale au niveau du côlon, comportant au moins comme premier probiotique des bifidobactéries sous forme vivante, et éventuellement un autre probiotique, **caractérisée en ce que,** dans le but d'assurer un taux de survie et d'adhérence spécifique des bifidobactéries au niveau du côlon, la concentration de la préparation en bifidobactéries est supérieure à 3 milliards de bifidobactéries par gramme de préparation, et en ce que les bifidobactéries sont doublement encapsulées, de manière à conserver leur intégrité au cours du transfert dans le tractus gastro-intestinal.

La concentration en probiotique et son conditionnement, conformes à la présente invention, permettent d'obtenir un effet optimal au niveau du côlon.

En effet, on connaît déjà des préparations destinées à être ingérées par les sujets, qui contiennent des souches de bifidobactéries. Par exemple, dans le brevet PCT WO2006013588, des souches bactériennes mutantes productrices d'acide folique sont incorporées dans un aliment ou une préparation. Ces lignées bactériennes produisent des quantités importantes d'acide folique, pour certaines sans rétrocontrôle, et quel que soit le pH de l'environnement. Dans cette application, le taux de survie dans le tractus gastro-intestinal des bactéries productrices d'acide folique n'est pas critique et, si les bactéries productrices d'acide folique doivent être qualitativement présentes, l'objectif n'est pas de modifier les proportions des populations bactériennes dans le côlon. La concentration en bactéries est de préférence entre 10⁹ et 10¹⁰ par g de produit final. Les germes utilisés dans le brevet WO2006013588 sont lyophilisés et ils ne sont pas encapsulés, ce qui n'est pas de nature à obtenir un taux de survie très élevé.

En effet, le stress et le déficit d'activité de souches bactériennes lyophilisées ont été mis en évidence par Gardiner et al. (Comparative Survival Rates of Human-Derived Probiotic Lactobacillus paracasei and L. salivarius Strains during Heat Treatment and Spray Drying, APPLIED AND ENVIRONMENTAL MICROBIOLOGY, June 2000, p. 2605-2612), ou par Corcoran et al. (Enhanced Survival of GroESL-Overproducing Lactobacillus paracasei NFBC 338 under Stressful Conditions Induced by Drying, APPLIED AND ENVIRONMENTAL MICROBIOLOGY, July 2006, p. 5104-5107). Ces deux références proposent des solutions pour diminuer ce stress, et la double encapsulation des probiotiques apporte également une grande amélioration.

D'une manière plus générale, on estime à 92% la perte de bactéries pendant la manipulation et le stockage d'un complément alimentaire à base de probiotiques, au bout de 3 mois, du fait de la température, de la pression, de l'humidité, ou du contact avec d'autres principes actifs.

On apprend du brevet US2008-069861 qu'un probiotique, dont la composition peut comporter des bifidobactéries ou des bacteroides, est associé à un glucide et à une protéine qui résistent à la digestion dans le petit intestin. C'est l'association des trois éléments qui a un effet sur la bonne santé du gros intestin. La concentration en probiotiques est entre 10⁴ et 10¹² CFU par 10g de composition, et les germes utilisés ne sont pas encapsulés. Comme pour le brevet WO2006013588, l'effet recherché n'est pas de modifier les proportions des populations bactériennes dans le côlon.

On apprend aussi du brevet US2007116826, que des probiotiques comportant en particulier des bifidobactéries sont incorporés dans des boissons de type soda, dont la caractéristique principale est par ailleurs le remplacement du sucre par des édulcorants. Comme pour les brevets précédents, l'effet recherché n'est pas de modifier les proportions des populations bactériennes dans le côlon.

Avec en vue les objectifs précédemment énoncés, le traitement par la présente préparation vise en revanche à modifier les proportions des populations bactériennes. C'est pourquoi la concentration en bifidobactéries doit être élevée dans le produit ingéré, et les probiotiques doivent être conditionnés pour survivre d'une part au stockage, et d'autre part au transit dans le tractus gastro-intestinal.

Selon une disposition particulière, les bactéries constituant le probiotique sont donc doublement encapsulées. En effet, pour être actives, les cellules bactériennes doivent avoir leur intégrité préservée tout au long du trajet dans le tractus gastro-intestinal. Cette double encapsulation permet de maintenir actives les bifidobactéries tout le long de ce trajet, jusqu'au niveau du côlon.

Le double encapsulation des bactéries, conformément au Brevet EP-1514553, est effectué selon le système connu sous le nom de « Duolac » (marque déposée). En effet, les probiotiques préparés selon la technologie « Duolac » sont doublement micro-encapsulés permettant ainsi de multiplier par 10 le nombre de bactéries atteignant vivantes l'intestin et garantir une régénération et un rééquilibrage du microbiote intestinal. Si l'on consomme des probiotiques classiques, un grand nombre de cellules viables est tué avant d'atteindre l'intestin à cause du pH acide de l'estomac et de l'hydrolyse enzymatique qui s'y produit. Le pH de l'estomac varie normalement d'un pH 2 (estomac vide) à un pH 4 (après le repas). Durant le transit qui peut durer 120 minutes, ce milieu acide tue plus de 90 % des micro-organismes vivants sauf si ceux-ci sont protégés. L'encapsulation est basée sur un système pH-dépendant qui protège la bactérie dans le milieu acide de l'estomac (pH 2 - pH 4) et la libère dans l'environnement neutre de l'intestin (pH 6 - pH 7). En effet, la première enveloppe est constituée de peptides et d'une matrice protéique dont l'ouverture est dépendante du pH. La seconde enveloppe est constituée de polysaccharides et d'hydrocolloïdes qui protègent les bactéries contre les autres facteurs physico-chimiques tels que la température, la pression ou l'humidité. Cette seconde enveloppe est surtout utile pendant la confection de la préparation. De plus, la double encapsulation protège les germes également contre les acides biliaires. La double encapsulation décrite permet de multiplier par 10 le nombre de bactéries atteignant vivantes le gros intestin. Enfin, la double encapsulation des bifidobactéries permet d'augmenter leur pouvoir d'adhérence aux cellules épithéliales intestinales. L'ensemble de ces observations peut être retrouvé dans le rapport intitulé « Safety study of Duolac » publié par CELL BIOTECH EUROPE le 8 novembre 2006.

Préférentiellement, le probiotique est sous une forme très concentrée, supérieure à 3 milliards de bifidobactéries par gramme de préparation, et préférentiellement supérieure à 30 milliards de bifidobactéries par gramme de préparation. Comme ces bactéries sont potentiellement très actives du fait qu'elles sont lyophilisées et du fait de leur mode de conservation, cette quantité initiale permet de générer dans l'intestin 3 x 10¹⁶ bactéries par heure après ingestion d'une prise de 10 g de préparation.

La préparation selon l'invention contient préférentiellement une quantité de fibres prébiotiques spécifiques capables de favoriser sélectivement certaines espèces non pathogènes da la famille de Bacteroides et plus particulièrement la population de Bacteroides thetaiotaomicron au niveau du côlon. La préparation selon l'invention peut aussi inclure directement dans sa composition des Bacteroides thetaiotaomicron.

Selon une autre disposition, la préparation selon l'invention ne contient pas de firmicutes, de manière à augmenter le rapport bactéroïdetes/firmicutes au niveau du côlon.

L'invention permet de résoudre les problèmes mentionnés ci-dessus. En effet, par son effet réparateur de la paroi intestinale, la préparation permet bien une diminution des phénomènes d'inflammation liés à l'obésité, et favorise la régulation de l'ingestion des aliments. Elle permet en particulier d'agir en même temps sur la prise de poids, les phénomènes inflammatoires et le déclenchement du diabète de type II.

L'invention permet de favoriser sélectivement certaines espèces non pathogènes de la famille de Bacteroides et plus particulièrement la population de Bacteroides thetaiotaomicron au niveau du côlon. Par l'absence de lactobacilles dans la composition de la préparation, l'invention permet d'atteindre ces objectifs en augmentant le rapport bactéroïdètes/firmicutes de la flore intestinale.

De plus, par l'utilisation de bifidobactéries, la préparation sera spécifiquement active au niveau du côlon, et présentera un effet positif sur l'hypercholestérolémie associée à l'obésité, par le fait que les bifidobactéries favorisent aussi la régulation du métabolisme lipidique.

Selon une disposition particulière de l'invention, la préparation est particulièrement destinée pour les adolescents et les adultes, et constitue un moyen d'équilibrer la flore intestinale déviante. Il est particulièrement recommandé pour les sujets ayant tendance à l'excès pondéral, et pour stabiliser le poids chez les sujets qui étaient en surpoids ou obèses, ayant perdu du poids volontairement à la suite d'un régime ou d'une intervention chirurgicale.

En effet, les inventeurs ont pu observer l'effet de traitements par la préparation sur des sujets adultes ou adolescents.

Le brevet WO-2008116700 préconisait déjà un traitement bifidogène de la femme enceinte ou du nourrisson. Ce traitement prévoit l'administration de souches de Bifidobacterium comme probiotiques, en association avec des sucres et des fibres non digérables, que le brevet ne considère pas comme prébiotiques mais comme des agents laxatifs chez le jeune enfant. La colonisation de l'intestin par des bifidobactéries dès le plus jeune âge est ici considérée comme un facteur de prévention du risque d'obésité ultérieure. WO-2006091103 propose une composition de Bifidobacterium Breve et éventuellement Lactobacillus paracasei en présence de 2 polysaccharides dont les caractéristiques sont affinées pour optimiser la croissance des bifidobactéries. La visée thérapeutique de cette préparation est la nutrition du jeune enfant non allaité, en vue de réduire les risques de désordres digestifs, de phénomènes allergiques et d'infection à court terme, et de diabète et d'obésité à plus long terme. Il s'agit surtout ici d'optimiser l'apport nutritionnel d'un nouveau-né qui n'est pas allaité. De même, le brevet EP1776877 vise la nutrition du nourrisson né par césarienne.

Mais on sait que le microbiote du nouveau-né et du jeune enfant est particulière, avec une prédominance marquée des bifidobactéries, qui représentent 42,2% des bactéries totales chez le nouveau-né (Violaine Rochet, Lionel Rigottier-Gois, Caractérisation moléculaire de la diversité et de la composition du microbiote digestif de l'homme par hybridation in situ fluorescente couplée à la cytométrie de flux. Unité d'Ecologie et Physiologie du Système Digestif, INRA Jouy-en-Josas, Nov. 2005). Chez le nourrisson, le rapport entre firmicutes et bactéroïdètes n'est plus que de 5 au lieu de 10 chez l'adulte sain ayant entre 20 et 50 ans (Mariat et al., The Firmicutes/Bacteroidetes ratio of the human microbiota changes with age. BMC Microbiology, 2009, 9 :123). Le microbiote du nouveau-né est donc particulière, et joue un rôle important dans la prévention de l'obésité et du diabète.

Cette remarque est d'ailleurs cohérente avec les recommandations du rapport intitulé « Alimentation infantile et modification de la flore intestinale » de l'Agence française de sécurité sanitaire des aliments (AFSSA, Juin 2003). Concernant « l'impact sur la santé des nourrissons et des jeunes enfants du développement de l'incorporation de prébiotiques et de probiotiques dans les aliments lactés diététiques qui leur sont spécifiquement destinés », l'AFSSA s'est fixé comme objectifs de « faire l'état des connaissances sur les questions posées par la modification de la flore intestinale chez les jeunes enfants, en examinant tous les aspects liés à l'utilisation des ingrédients modifiant la flore intestinale dans les préparations pour nourrissons », et « d'élaborer des lignes directrices pour la constitution de dossiers industriels spécifiques à ces types de produits ».

Rien ne montrait donc que le contexte du microbiote du jeune enfant ou du nourrisson aurait pu être transposable à l'adolescent ou à l'adulte.

Préférentiellement, les fibres prébiotiques utilisées sont des fibres à effet bifidogène, et comportent au moins des galacto-oligosaccharides et de l'oligo-fructose. Les fibres bifidogènes amplifient ainsi l'effet des bifidobactéries en permettant leur multiplication dans l'intestin et favorisent sélectivement certaines espèces non pathogènes de la famille de Bacteroides et plus particulièrement la population de Bacteroides thetaiotaomicron au niveau du côlon.

Préférentiellement aussi, l'agent actif facilitant l'action des probiotiques est un régénérateur de la paroi intestinale, par exemple la L-glutamine, qui a une action réparatrice sur la perméabilité de la paroi intestinale. Cette action ne peut donc que renforcer celle des bifidobactéries qui ont été choisies pour leur contribution à cette réparation.

Selon une autre disposition de l'invention, l'effet positif de la préparation sur la santé sera renforcé par l'incorporation dans la préparation d'extraits d'origine végétale ou d'agents phytothérapeutiques, et des bactéries commensales de l'Homme.

D'autres caractéristiques et avantages apparaîtront dans la description qui va être faite d'un exemple de réalisation de l'invention.

Dans cet exemple, la préparation selon l'invention se présente sous une forme générale, connue de l'homme de l'art, permettant d'assurer la survie du probiotique. Les bactéries probiotiques utilisées pour la mise en oeuvre de la présente invention sont du genre bifidibacterium. Selon une mise en oeuvre préférée de l'invention les bactéries probiotiques sont:
Bifidobacterium bifidum BF2,
Bifidobacterium longum BG3,
Bifidobacterium breve BR2,
Bifidobacterium infantis BT1,
Bifidobacterium lactis BL2.

Dans le choix d'un probiotique pour obtenir la préparation selon l'invention il est fondamental de rechercher des bactéries de préférence connues comme ayant de bonnes propriétés en ce qui concerne leur taux de survie dans le tractus digestif et notamment qui ne soient pas détruites par les acides gastriques et biliaires. Dans la mesure où elles présentent ces différents avantages mentionnés ci-avant les bactéries probiotiques, Bifidobacterium bifidum BF2, Bifidobacterium longum BG3, Bifidobacterium breve BR2, Bifidobacterium infantis BT1, Bifidobacterium lactis BL2 sont particulièrement préférées. Les souches sélectionnées présentent une adhésion accrue aux cellules épithéliales intestinales, comme cela a été démontré dans la publication intitulée « Adhesion Efficiency of Cell Biotech Strains to the Intestinal Epithelial Cells », publiée par the Research Institute of Cell Engineering, Cell Biotech Co., Ltd, le 15 novembre 2006. Les probiotiques sont doublement encapsulés, selon la description qui en a été précédemment faite, ce qui augmente encore le pouvoir d'adhésion de ces micro-organismes aux cellules épithéliales intestinales.

La préparation selon l'invention comprend également préférentiellement un prébiotique qui est une substance qui ne peut-être digérée par le patient et qui stimule la croissance ou l'activité de certaines bactéries non pathogènes présentes dans le tractus gastro-intestinal, ou facilite la survie du probiotique administré simultanément. Les fibres prébiotiques utilisées pour la mise en oeuvre de l'invention sont des oligosaccharides, et plus particulièrement des transgalacto-oligosaccharides ou des oligo-fructose.

La préparation de l'invention comprend aussi préférentiellement un agent actif facilitant l'action des probiotiques au niveau de la paroi intestinale; préférentiellement ce potentialisateur est un acide aminé et plus préférentiellement encore de la L-glutamine.

La préparation selon l'invention peut encore avantageusement contenir au moins un acide gras essentiel, des extraits végétaux, des composés phytothérapeutiques et des bactéries commensales de l'Homme améliorant l'effet sur la santé générale du sujet. Comme composés phytothérapeutiques, la préparation selon l'invention pourrait comprendre la pulpe fraîche de la feuille interne d'Aloe Vera variété Barbadensis Miller et de la racine du Curcuma Longa fermenté.

Comme bactéries commensales de l'Homme, la préparation selon l'invention pourrait comprendre l'espèce de Bacteroides thetaiotaomicron.

La préparation selon l'invention peut se présenter sous toute forme appropriée, connue de l'homme de l'art, adaptée notamment pour assurer la survie des bactéries. Il est important de tenir compte du facteur température. Il est donc impératif, afin de maintenir les bactéries vivantes, qu'elles soient conservées entre 4 et 8°C (APPLIED AND ENVIRONMENTAL MICROBIOLOGY, June 2000, p.2605-2612). Le respect de cette chaîne du froid doit être garanti dans le but de proposer aux consommateurs des probiotiques optimaux. Cette conservation au frais s'applique pendant le transport, le stockage et la conservation du produit.

De préférence la préparation de l'invention est sous forme de poudre.

L'exemple non exhaustif et non limitatif ci-après permet d'illustrer l'invention :
Les bactéries probiotiques utilisées sont celles commercialisées sous la marque « Duolac » ™.

Le mélange suivant est réalisé à une température comprise entre 4-8°C et sous atmosphère stérile :
- 66,7 g de prémix de bifidobactéries « Duolac »™
   B. bifidum BF2 (3E+9 cfu/g)
   B. longum BG3 (3E+9 cfu/g)
   B. breve BR2 (3E+9 cfu/g)
   B. infantis BT1 (1,05E+10cfu/g)
   B. lactis BL2 (1.05E+10 cfu/g)
   Total vcc. 3E+E10 cfu/g (BF2, BG3, BR2, BT1 et BL2)
- 820g de fibres prébiotiques dont :
   700 g de trans-galactooligosaccharides et
   100 g d' oligo-fructose
- 100g de potentialisateur (L-glutamine)

Le mélange obtenu sous forme de poudre est réparti en sachets monodoses de 5 ou 10 grammes.

## Revendications

1. Préparation destinée à corriger une dysbiose intestinale au niveau du côlon, comportant au moins comme premier probiotique des bifidobactéries sous forme vivante, et éventuellement un autre probiotique, **caractérisée en ce que,** dans le but d'assurer un taux de survie et d'adhérence spécifique des bifidobactéries au niveau du côlon, la concentration de la préparation en bifidobactéries est supérieure à 3 milliards de bifidobactéries par gramme de préparation, et **en ce que** les bifidobactéries sont doublement encapsulées, de manière à conserver leur intégrité au cours du transfert dans le tractus gastro-intestinal.

2. Préparation selon la revendication 1 **caractérisée en ce que** le premier probiotique comporte le mélange des bifidobactéries :Bifidobacterium bifidum BF2, Bifidobacterium longum BG3, Bifidobacterium breve BR2, Bifidobacterium infantis BT1, Bifidobacterium lactis BL2.

3. Préparation selon la revendication 1, **caractérisée en ce qu'**elle comporte préférentiellement plus de 30 milliards de bifidobactéries par gramme de préparation.

4. Préparation selon la revendication 1 **caractérisée en ce que** le probiotique est sous forme de poudre lyophilisée.

5. Préparation selon la revendication 1 **caractérisée en ce que** le second probiotique comporte des espèces non pathogènes de la famille de Bacteroides et plus particulièrement Bacteroides thetaiotaomicron.

6. Préparation selon la revendication 1 **caractérisée en ce qu'**elle contient en outre des fibres prébiotiques, en particulier des fibres prébiotiques à effet bifidogène, qui favorisent en outre des espèces non pathogènes de la famille de Bacteroides et plus particulièrement la population de Bacteroides thetaiotaomicron au niveau du côlon.

7. Préparation selon l'une quelconque des revendications 1 à 6 **caractérisée en ce qu'**elle contient pas de firmicutes.

8. Préparation selon la revendication 6 **caractérisée en ce que** les fibres prébiotiques comportent des oligosaccharides, en particulier des transgalacto-oligosaccharides ou des oligo-fructoses.

9. Préparation selon la revendication 1 contenant en outre un agent actif facilitant l'action des probiotiques par un effet régénérateur de la paroi intestinale, en particulier de la L-Glutamine.

10. Préparation selon la revendication 1 **caractérisée en ce qu'**elle comporte au moins un acide gras essentiel.

11. Préparation selon la revendication 1 **caractérisée en ce qu'**elle comporte des agents phytothérapeutiques et des bactéries commensales de l'Homme, connues pour leur effet positif sur la santé générale du sujet.

12. Préparation selon l'une quelconque des revendications 1 à 11, pour son utilisation dans le traitement de l'obésité et des désordres physiologiques qui lui sont associés, par son action sur l'excès pondéral.

13. Préparation pour son utilisation selon la revendication 12 **caractérisée en ce qu'**elle est consiste en un médicament, un complément alimentaire, un aliment, ou un additif alimentaire.

14. Préparation pour son utilisation selon la revendication 12 destinée au traitement chez l'adolescent et l'adulte de l'excès pondéral ou de l'obésité ou du diabète de type II ou de l'hypercholestérolémie.

15. Préparation pour son utilisation selon la revendication 12 pour stabiliser le poids chez des sujets ayant été en surpoids ou obèses, ayant perdu du poids volontairement à la suite d'un régime ou d'une intervention chirurgicale.

16. Procédé de fabrication de la préparation selon l'une quelconque des revendications 1 à 15 **caractérisé en ce que** le probiotique sous forme de poudre lyophilisée est conservé à basse température grâce à une chaîne de froid qui permet de conserver un maximum de micro-organismes vivants tout au long de la vie de la dite préparation.
